(19) Europäisches Patentamt
European Patent Office
Office européen des brevets

(11) Publication number: **0 050 002**
**B1**

(12) # EUROPEAN PATENT SPECIFICATION

(45) Date of publication of patent specification: **10.07.85**

(51) Int. Cl.⁴: **C 07 D 277/14,** A 61 K 31/425

(21) Application number: **81304653.9**

(22) Date of filing: **07.10.81**

(54) **2-Substituted-phenyl-5-alkylthiazolidin-4-ones for peptic ulcer treatment.**

(30) Priority: **09.10.80 JP 141239/80**

(43) Date of publication of application:
**21.04.82 Bulletin 82/16**

(45) Publication of the grant of the patent:
**10.07.85 Bulletin 85/28**

(84) Designated Contracting States:
**CH DE FR GB IT LI**

(56) References cited:
**FR-A-2 329 662**
**GB-A-1 345 159**
**GB-A-2 055 816**
**US-A-2 623 048**
**US-A-4 053 471**

**CHEMICAL ABSTRACTS, vol. 89, no. 13, 25th September 1978, page 71, no. 100017s Columbus, Ohio, U.S.A. A. TEROL et al.: "Study on 2-phenylthiazolidine derivatives as radioprotectant agents"**

(73) Proprietor: **KUREHA KAGAKU KOGYO KABUSHIKI KAISHA**
**9-11 Horidome-cho 1-chome Nihonbashi Chuo-ku**
**Tokyo (JP)**

(72) Inventor: **Kawasaki, Takao**
**532-27 Mizuno**
**Sayama-shi Saitama-ken (JP)**
Inventor: **Osaka, Yoshiaki**
**5-672-9 Matsugaoka**
**Nagareyama-shi Chiba-ken (JP)**
Inventor: **Komatsu, Katsumi**
**3-1119 Matsudo Matsudo-shi**
**Chiba-ken Tokyo (JP)**
Inventor: **Yamaguchi, Yukiharu**
**1-13-1203 Tamazutsumi**
**Setagaya-ku Tokyo (JP)**
Inventor: **Ono, Saichi**
**5-31-1 Matsubara**
**Setagaya-ku Tokyo (JP)**

(74) Representative: **Myerscough, Philip Boyd et al**
**J.A.Kemp & Co. 14, South Square Gray's Inn**
**London, WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 050 002 B1**

Courier Press, Leamington Spa, England.

**Description**

The present invention concerns 2-(substituted phenyl)-5-alkylthiazolidin-4-ones which are useful as anti-peptic ulcer medicines, their preparation and pharmaceutical compositions containing them.

Peptic ulcers are collapsed, weakened parts of the gastric or enteric mucosa caused by the action of aggressive factors such as hydrochloric acid and pepsin in the gastric juice. Mild cases of peptic ulcer are curable after 3 to 4 months of hospitalization and treatment. However, serious cases are accompanied by haemorrhaging and perforation of the organ causing the problem to become chronic.

Abnormalities in the autonomic nerve system and in the mucosal blood flow due to physical and/or mental stress have been considered as etiological causes of peptic ulcers. However, it is practically impossible to interpret the etiology of peptic ulcer unitarily because the viscera themselves are subjected to complicated control by nerves and hormones.

Sodium hydrogen carbonate, aluminum salts and magnesium salts have been used for a long time as anti-peptic ulcer medicines as a means of neutralizing the hydrochloric acid in the gastric juice. However, these medicines only temporarily neutralize the acid to alleviate the pain and do not accelerate a substantial cure of the ulcer.

Recently, many kinds of anti-ulcer medicines have been developed based on presumed causes of ulcers, including the medicines for suppression of the autonomic nerve, that is, so-called anti-cholinergic agents, agents for repairing the damaged tissues and agents for improving the blood flow. However, the present situation is that none of them can be said to be satisfactory in view of their ineffectiveness or their side effects.

For instance, carbenoxolone which has been commercialized as an anti-peptic ulcer medicine has been broadly used because of its excellent effect in accelerating the cure of ulcers. However, it has aldosterone-like side effects which cause hypertension and weakening of muscular functions when it is taken continuously. The anti-cholinergic agents show severe side effects such as mydriasis and thirst due to the blocking of the parasympathetic nerve, and it has been reported their effects of accelerating the ulcer-curing is low.

Since it generally takes a long time to cure a peptic ulcer, the period of administration of an anti-peptic ulcer medicine extends from 100 to 150 days on average and from one to two years in some cases. Accordingly, it is required that an anti-peptic ulcer medicine should be very safe as well as highly effective in curing ulcers.

The compounds according to the present invention are 2-(substituted phenyl)-5-alkylthiazolidin-4-ones represented by the following formula:

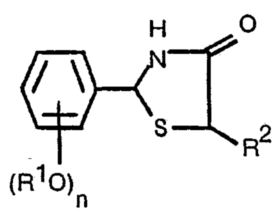

(I)

wherein $R^1$ and $R^2$ independently represent an alkyl group of from 1 to 3 carbon atoms, and n is an integer of from 1 to 3.

2-Substituted-phenyl-5-alkylthiazolidin-4-ones represented by formula (I) have excellent anti-peptic ulcer action and are pharmacologically safe compounds. 2-Substituted-phenyl-5-alkylthiazolidin-4-ones according to the present invention (hereinafter referred to as the present compounds) include the following compounds:

2-(2,3,4-trimethoxyphenyl)-5-methylthiazolidin-4-one,
2-(3,4,5-trimethoxyphenyl)-5-methylthiazolidin-4-one,
2-(2,3-dimethoxyphenyl)-5-methylthiazolidin-4-one,
2-(2,4-dimethoxyphenyl)-5-methylthiazolidin-4-one,
2-(2,5-dimethoxyphenyl)-5-methylthiazolidin-4-one,
2-(2,6-dimethoxyphenyl)-5-methylthiazolidin-4-one,
2-(3,4-dimethoxyphenyl)-5-methylthiazolidin-4-one,
2-(3,5-dimethoxyphenyl)-5-methylthiazolidin-4-one,
2-(2-methoxyphenyl)-5-methylthiazolidin-4-one,
2-(3-methoxyphenyl)-5-methylthiazolidin-4-one, and
2-(4-methoxypheny)-5-methylthiazolidin-4-one.

The melting points, appearances and elementary analytical compositions of some of the present compounds are shown in Table 1.

TABLE 1

| Compound number | Name of compound | Structural formula | Melting point (°C) |
|---|---|---|---|
| 1 | 2-(3,4,5-trimethoxyphenyl)-5-methylthiazolidin-4-one | 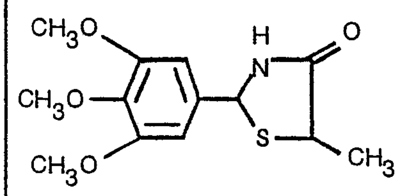 | 133 – 134 |
| 2 | 2-(3,4-dimethoxyphenyl)-5-methylthiazolidin-4-one | 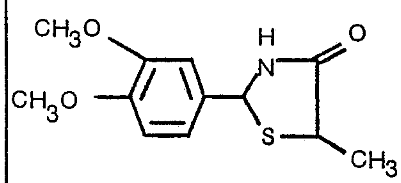 | 151 – 152.5 |
| 3 | 2-(2-methoxyphenyl)-5-methylthiazolidin-4-one | 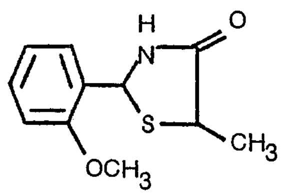 | 187 – 187.5 |
| 4 | 2-(4-methoxyphenyl)-5-methylthiazolidin-4-one | 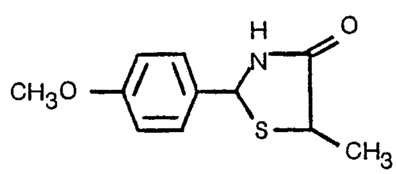 | 129 – 130 |

TABLE 1 (Continued)

| Compound number | Appearance | Elementary analytical composition (%) C | H | N | S |
|---|---|---|---|---|---|
| 1 | colourless aciculate | 55.09 (55.11) | 6.07 (6.05) | 4.93 (4.94) | 11.27 (11.31) |
| 2 | colourless leaf-like | 56.89 (56.90) | 5.99 (5.97) | 5.53 (5.53) | 12.62 (12.66) |
| 3 | colourless prism | 59.14 (59.17) | 5.88 (5.87) | 6.25 (6.27) | 14.39 (14.36) |
| 4 | colourless minute aciculate | 59.17 (59.17) | 5.85 (5.87) | 6.29 (6.27) | 14.40 (14.36) |

Note: The parenthesized figures in the Elementary Analytical Composition column show the theoretical values based on the molecular formula of each compound.

The present compound may be produced by either of the following two methods (1) and (2):

(1) A process which comprises reacting an aldehyde represented by the formula (II):

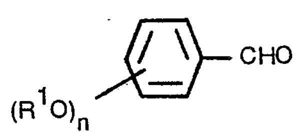

(II)

wherein $R^1$ represents an alkyl group of 1 to 3 carbon atoms and n is an integer of 1 to 3, with a compound of the formula (III) $R^2$—CH(SH)—$COR^3$ wherein $R^2$ is an alkyl group of 1 to 3 carbon atoms and $R^3$ represents a hydroxyl group or alkoxy group of 1 or 2 carbon atoms, preferably 2-mercapto-propionic acid or the methyl or ethyl ester thereof, and an ammonium compound in an inert solvent such as benzene, toluene and xylene. Preferably an equimolar amount or a slight excess of the compound of formula (III) is used. The ammonium compound, preferably ammonium carbonate, is preferably used in such an amount that there

is a slight excess as ammonia. The reaction can be carried out at a temperature of 50 to 150°C, usually at the boiling point of the solvent, for 1 to 10 hours.

The method (1) can be summarized as follows:

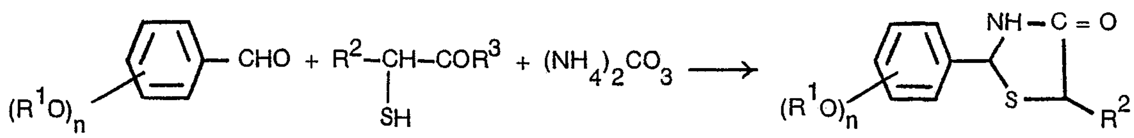

wherein $R^1$ and $R^2$ each represent an alkyl group with 1 to 3 carbon atoms, $R^3$ represents a hydroxyl group or an alkoxy group with 1 or 2 carbon atoms, and n is an integer of 1 to 3.

(2) A process which comprises reacting an aldehyde represented by the formula (II) with a compound represented by the formula (IV) $R^2$—CH(SH)—$CONH_2$ wherein $R^2$ is an alkyl group of 1 to 3 carbon atoms, preferably 2-mercaptopropionamide, in an inert solvent such as benzene, toluene and xylene. Preferably an equi-molar amount or slight excess of the compound of formula (IV) is used. The reaction can be carried out at a temperature of 50 to 150°C, usually at the boiling point of the solvent, for 1 to 3 hours.

No matter which method may be adopted, on cooling the reaction mixture after the reaction is over, the desired product separates out as crystals. The crystals can be collected by filtration. They are purified by recrystallization, using a solvent ordinarily used for recrystallization such as benzene, methanol or ethanol.

The important problem in the development of an anti-peptic ulcer medicine is the screening of the medicine. Hitherto, the evaluation of anti-ulcer medicines has been frequently carried out based on their prophylactic effect against the acute ulcers such as ulcers due to pyloric ligation, aspirin or indomethacin. However, to what extent the results of evaluations by these models reflect the effect of the medicines on human ulcers has not been fully elucidated.

The inventors of the present invention, taking into account this situation, included with the above-mentioned evaluation methods a technique based on the oral administration of the present compounds and a commercial anti-peptic ulcer medicine, respectively, to rats in which duodenal peptic ulcers due to acetic acid (refer to Okabe, 1971), considered to most closely resemble human peptic ulcers, have been artificially formed.

Anti-peptic ulcer effect of the present compound:

(1) Effect on peptic ulcer due to pyloric ligation

Six groups (10 animals per group) of male rats weighing 180 to 200 g were subjected to ligation of their pylori under ether-anesthesia after fasting for 48 hours according to the method of Shay *et al.* (refer to Gastroenterology, *5*, page 43 (1945)).

Just after ligation, one of the present compounds suspended in an aqueous physiological saline solution was intraperitoneally administered to a rat. Into the rats of one control group an aqueous physiological saline solution was injected, whilst into the rats of another control group Gefarnate was injected. Then, after 15 hours of fasting without water, the rats were sacrificed with ether. Their stomachs were removed for examination under a microscope. The length and width of the thus-formed ulcer in the stomach were determined and their product calculated ($mm^2$). The total sum of the products represented the ulcer coefficient. The results are shown in Table 2.

TABLE 2

| Compound number | Dose rate (mg/kg) | Ulcer coefficient ($mm^2$) | Rate of suppression[1]) (%) |
|---|---|---|---|
| 1 | 100 | 7.2 | 82.5 |
| 2 | 100 | 6.5 | 84.2 |
| 3 | 100 | 3.6 | 91.2 |
| 4 | 100 | 4.1 | 90.0 |
| Positive control[2]) | 100 | 36.1 | 12.2 |
| Control | – | 41.1 | 0 |

Notes [1]) Rate of suppression of ulcer =

$$\frac{\text{Ulcer coefficient (control)} - \text{Ulcer coefficient (treated group)}}{\text{Ulcer coefficient (control)}} \times 100$$

[2]) Positive control: Gefarnate = 3,7-dimethyl-2,6-octadienyl 5,9,13-trimethyl-4,8,12-tetradecatrienoate

As shown in Table 2, the present compounds showed a rate of suppression of the occurrence of peptic ulcer of 83 to 91% at an intraperitoneal administration of 100 mg/kg body weight, whereas the commercial anti-peptic ulcer medicine, gefarnate, showed a suppression rate of only about 12% at the same dose level.

(2) Effect on peptic ulcer due to acetic acid

Following the method of Okabe *et al* (refer to Amer. J. Dig. Dis., 16, (1977)), 6 groups (15 animals per group) of male rats weighing 240 to 260 g were subjected to laparotomy under ether anesthesia in which a metal circular frame was plaed on the serosa at a distance of 5 to 7 mm from the duodenal pylorus, and 0.06 ml of glacial acetic acid was poured into the frame. After 30 seconds, the liquid containing the acetic acid was removed and then the frame was removed. One test compound suspended in an aqueous physiological saline solution was orally administered to a rat 3 times a day from the third day after the operation for consecutive 10 days. After the administration was over, the rats were sacrificed with ether, and their duodenum was removed for observation under a microscope. The length and width of the thus-formed ulcer were measured and their product (expressed with $mm^2$) was recorded as the ulcer coefficient. The results are shown in Table 3.

TABLE 3

| Compound number | Dose rate (mg/kg) | Ulcer coefficient ($mm^2$) | Rate of suppression of ulcer (%) |
|---|---|---|---|
| 1 | 100 | 2.8 | 77.5 |
| 2 | 100 | 2.4 | 70.0 |
| 3 | 100 | 1.3 | 83.8 |
| 4 | 100 | 1.5 | 81.3 |
| Positive control [1]) | 100 | 6.4 | 20.0 |
| Control | – | 8.0 | 0 |

Note: [1]) Positive control: gefarnate (refer to the footnote of Table 2)

Using this method of evaluation, effectiveness is not seen with antiacid and anti-cholinergic medicines, both of which have been conventionally used as anti-ulcer medicines, and only a slight effectiveness is recognized in respect of gefarnate which is intended to repair damaged tissue. On the other hand, in the group of rats administered with the present compound, a remarkable curative effect was recognized. Even on histological observation of the ulcer lesion, a state of complete cure had been obtained.

This experimental model has been highly evaluated internationally because the thus-formed ulcer is scarcely curable in nature and the histopathological change of the ulcer lesion closely resembles that of human chronic ulcers as compared to the method of cautery-ulcer (refer to Skoryna, 1958) and the method of crumping-cortisone (refer to Umehara, 1965).

(3) On evaluation by the hitherto broadly utilized effective methods for screening anti-peptic ulcer medicines such as those based on stress, aspirin and indomethacin-induced ulcers, the present compounds showed effects superior to the effects of commercial anti-peptic ulcer medicines.

Toxicological properties of the present compound:

(1) Acute toxicity test

Experimental animal:

Female ICR-mice of body weight of 20 to 24 g, 5 weeks after birth were used.

Method of rearing:

Eight animals per group were kept in a transparent polycage at room temperature of 23 ± 1°C, and RH of 60 to 70%.

Administration of the present compound:

After minutely pulverizing each one of the present compounds, a pulverized compound was suspended in an aqueous 5% sodium carboxy-methylcellulose solution containing 20% of Tween-80®. The aqueous suspension was forcibly orally administered by a metal stomach tube, the dose rate having been adjusted by changing the concentration of the present compound in the aqueous suspension.

General symptoms due to administration of the present compound:

In cases of administration at higher dose rates, the rats became inactive but, after 2 to 3 hours, they became normal. In some cases where death occurred, spontaneous movement was reduced with reduction of general tension and the rats died as they were.

Calculation of $LD_{50}$:

The rats' mortality was observed for a week after the administration. The $LD_{50}$ was calculated from the mortality rate by the Litchfield-Wilcoxon formula. The results are shown in Table 4.

TABLE 4

| Compound number | $LD_{50}$ (p.o.) (mg/kg) |
|---|---|
| 1 | 6400 |
| 2 | more than 8000 |
| 3 | more than 8000 |
| 4 | more than 8000 |

In addition, according to the results of acute toxicity tests using rats and mice as experimental animals, $LD_{50}$ i.v. was larger than 1.2 g/kg.

(2) Sub-acute toxicity test

Experimental animal:

Both sexes of Sprague-Dawley rats of 110 to 150 g of body weight were used 5 months after birth.

Method of rearing:

Each five males and five females were respectively kept in a metal wire-net cage at room temperature of 22 to 24°C and RH of 60 to 70% for 3 months, each experimental group consisting of 10 males or 10 females.

Administration of the present compound:

Compound No. 2 of the present compounds, 2-(3,4-dimethoxyphenyl)-5-methylthiazolidin-4-one, was minutely pulverized and mixed with the powdery diet for the rats at a concentration of 0.4% by weight. The thus prepared diet was taken *ad lib.* The mean intake of the present compound was 400 mg/kg/day.

Examination:

The diet intake and the body weight of each rat were measured every other day and once a week, respectively. Urinalysis for glucose, protein, pH, and occult blood was carried out once a month. Blood samples were examined after ending the rearing. After sacrificing all the animals, they were autopsied to examine for the presence of abnormalities. Their organs were fixed with formaldehyde and embedded in paraffin to prepare sliced specimens of tissues stained with hematoxylineosine for microscopic observation.

Results:

(a) Diet intake was normal without a significant difference between experimental groups and the control group.

(b) Body weight gain was normal without significant difference between experimental groups and the control group.

(c) Mortality, (d) urinalysis, (e) hematological examination, and (f) findings on autopsy and histological examination were all normal without any significant difference between experimental groups and the control group.

Further, in sub-acute toxicity tests using mice as experimental animals, abnormal findings attributable to the present compound were never obtained.

As can be seen, the present compounds are very safe for administration. Accordingly, they can be used as an anti-peptic ulcer medicine for humans. In addition to its excellent pharmacological effects and toxicological properties, every compound of the present invention is colourless and crystalline, and almost all of them are tasteless or are only slightly bitter. Furthermore, since they are extremely stable without any change after storing at room temperature in an open state, their adaptability as an anti-peptic ulcer medicine can be said to be remarkably high.

The present invention provides a pharmaceutical composition comprising a compound of formula (I) as active ingredient together with a pharmaceutically acceptable carrier. Pharmaceutical compositions according to the present invention are useful for treatment of peptic ulcers and can comprise in unit dosage form a therapeutically effective amount of a compound of formula (I) together with a pharmaceutically acceptable carrier. The pharmaceutical composition in unit dosage form can be, for example, tablets, sugar-coated tablets, pills, capsules, powders, granules, troches, liquids, suppositories, injections, etc.

As the carrier, lactose, sucrose, sorbitol, mannitol, potato-starch, corn-starch, amylopectin, various kinds of starch, derivatives of cellulose (for instance carboxymethylcellulose and methylcellulose), gelatin, magnesium stearate, calcium stearate, polyvinyl alcohol, polyethylene glycol waxes, gum arabic, talc, titanium dioxide, vegetable oils such as olive oil, peanut oil and sesame oil, paraffin oil, neutral fatty bases, ethanol, aqueous physiological saline solutions, sterilized water, glycerol, colouring agents, flavourings, thickening agents, stabilizers, isotonic agents and buffering agents can be exemplified.

The content of the one of the present compounds in a pharmaceutical composition according to the invention is preferably 0.1 to 90% by weight, more preferably 1 to 60% by weight of the preparation.

The clinical daily dose of the present compound can be 60 to 6000 mg/60 kg of body weight, preferably, 150 to 3000 mg/60 kg body weight. The route of administration may be oral or by injection, preferably orally in the case of long term administration.

The following Examples illustrate the present invention. Throughout the description percentages are by weight unless otherwise specified.

Synthetic examples of the present compounds:

Example 1

Synthesis of 2-(3,4,5-trimethoxyphenyl)-5-methylthiazolidin-4-one

A mixture of 19.6 g of 3,4,5-trimethoxy-benzaldehyde, 10.6 g of 2-mercaptopropionic acid and 6 g of ammonium carbonate in 250 ml of benzene was refluxed for 3 hours at 80°C in a flask provided with a Dean-Stark apparatus and the thus distilled water was removed. The crystals which separated out from the reaction mixture after reaction was over and on cooling the reaction mixture, were collected by filtration and recrystallized from benzene to give 22.2 g of colourless and aciculates melting at 133 to 134°C in a yield of 78%.

Example 2

Synthesis of 2-(3,4-dimethoxyphenyl)-5-methyl-thiazolidin-4-one

A mixture of 16.6 g of 2,4-dimethoxy-benzaldehyde, 10.6 g of 2-mercaptopropionic acid and 6 g of ammonium carbonate in 250 ml of benzene was refluxed for 3 hours in a reaction vessel provided with a Dean-Stark apparatus at 80°C while removing the distilled water. The crystals which separated out after cooling the reaction mixture were collected by filtration and recrystallized from hot benzene to obtain the desired compound as colourless leaf-like crystals melting at 151 to 152.5°C in an amount of 20.4 g corresponding to a yield of 81%.

Example 3

Synthesis of 2-(2-methoxyphenyl)-5-methyl-thiazolidin-4-one

A mixture of 13.6 g of o-anisaldehyde, 13.4 g of ethyl 2-mercaptopropionate and 6 g of ammonium carbonate in 250 ml of toluene was refluxed at 110°C for 2 hours in a flask provided with a Dean-Stark apparatus while removing the distilled water. The crystals which separated out after leaving the reaction mixture for a night were collected by filtering and recrystallized from hot benzene to obtain the desired compound as colourless prisms melting at 187 to 187.5°C in an amount of 21 g corresponding to a yield of 94%.

Example 4

Synthesis of 2-(4-methoxyphenyl)-5-methyl-thiazolidin-4-one

A mixture of 13.6 g of p-anisaldehyde and 10.5 g of 2-mercaptopropionic acid in 150 ml of benzene was heated at 80°C for 2 hours. The crystals which separated out after leaving the reaction mixture for a night were collected by filtration and recrystallized from hot benzene to obtain colourless minute aciculates melting at 129 to 130°C in an amount of 20.5 g corresponding to a yield of 92%.

Manufacture of the pharmaceutical preparations:

Example 5

Manufacture of a granular preparation for oral administration:

Two hundred grams of 2-(3,4-dimethoxyphenyl)-5-methylthiazolidin-4-one was minutely pulverized and 800 g of corn-starch was admixed with the pulverized compound. After stirring the mixture well, 80 ml of an aqueous solution containing 3 g of sodium carboxymethyl-cellulose dissolved therein was added to the mixture. After kneading the whole mixture, it was fed to an extruding pelletizer to form granules. The granules were dried at a temperature of 60 to 80°C and screened to obtain the granular preparation for oral administration.

**Claims**

1. A compound which is a 2-(substituted phenyl)-5-alkylthiazolidin-4-one having the formula (I)

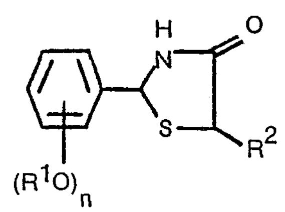

(I)

wherein $R^1$ and $R^2$ independently represent an alkyl group of from 1 to 3 carbon atoms and n denotes an integer of from 1 to 3.

2. A compound according to claim 1 having the formula

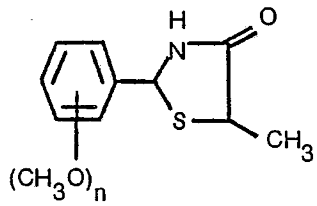

wherein n is an integer of 1 to 3.

3. A compound according to claim 1 which is 2-(3,4-dimethoxyphenyl)-5-methylthiazolidin-4-one.

4. A compound according to claim 1 which is 2-(3,4,5-trimethoxyphenyl)-5-methylthiazolidin-4-one.

5. A compound according to claim 1 which is 2-(2-methoxyphenyl)-5-methylthiazolidin-4-one.

6. A compound according to claim 1 which is 2-(4-methoxyphenyl)-5-methylthiazolidin-4-one.

7. A process for producing a compound of formula (I) as defined in claim 1, which process comprises reacting an aldehyde represented by the formula (II):

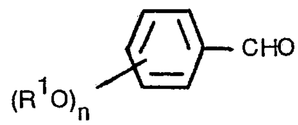

(II)

wherein $R^1$ and n are as defined in claim 1, with a compound of the formula $R^2$—CH(SH)—$COR^3$ wherein $R^2$ is as defined in claim 1 and $R^3$ represents a hydroxyl group or an alkoxy group having 1 or 2 carbon atoms and an ammonium compound in an inert solvent.

8. A process according to claim 7, wherein the reaction is carried out at a temperature of 50 to 150°C.

9. A process according to claim 7 or 8 wherein the ammonium compound is ammonium carbonate.

10. A process for preparing a compound of formula (I) as defined in claim 1, which process comprises reacting an aldehyde represented by the formula (II):

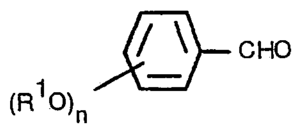

(II)

wherein $R^1$ and n are as defined in claim 1, with a compound represented by the formula $R^2$—CH(SH)—$CONH_2$ wherein $R^2$ is as defined in claim 1, in an inert solvent.

11. A process according to claim 10, wherein the reaction is carried out at a temperature of 50 to 150°C.

12. A pharmaceutical composition comprising a compound of formula (I) as claimed in any one of claims 1 to 6 or which has been produced by a process as claimed in any one of claims 7 to 11 as active ingredient, together with a pharmaceutically acceptable carrier.

13. A pharmaceutical composition suitable for treatment of peptic ulcer, which composition comprises in unit dosage form a therapeutically effective amount of a compound of formula (I) as defined in claim 1, and a pharmaceutically acceptable carrier.

**Patentansprüche**

1. 2-(Phenyl-substituiertes)-5-alkylthiazolidin-4-on der Formel I

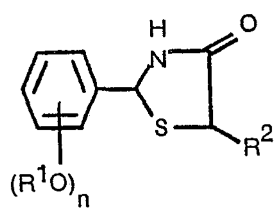

(I)

worin $R^1$ und $R^2$ unabhängig jeweils einen Alylrest mit 1 bis 3 Kohlenstoffatomen darstellen und n eine ganze Zahl von 1 bis 3 bedeutet.

2. Verbindung nach Anspruch 1 der Formel

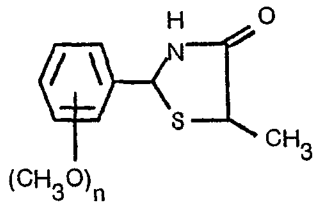

worin n eine ganze Zahl von 1 bis 3 bedeutet.

3. 2-(3,4-Dimethoxyphenyl)-5-methylthiazolidin-4-on.

4. 2-(3,4,5-Trimethoxyphenyl)-5-methylthiazolidin-4-on.

5. 2-(2-Methoxyphenyl)-5-methylthiazolidin-4-on.

6. 2-(4-Methoxyphenyl)-5-methylthiazolidin-4-on.

7. Verfahren zur Herstellung einer Verbindung der Formel I gemäß der Definition in Anspruch 1, wobei ein Aldehyd der Formel II

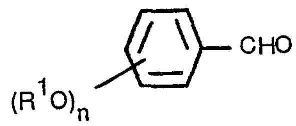

(II)

worin $R^1$ und n die in Anspruch 1 angegebene Bedeutung haben, mit einer Verbindung der Formel $R^2$—CH(SH)—$COR^3$, worin $R^2$ die in Anspruch 1 angegebene Bedeutung hat und $R^3$ eine Hydroxylgruppe oder eine Alkoxygruppe mit 1 bis 2 Kohlenstoffatomen darstellt und eine Ammonium-verbindung in einem inerten Lösungsmittel umgesetzt wird.

8. Verfahren nach Anspruch 7, worin die Reaktion bei einer Temperatur von 50 bis 150°C durchgeführt wird.

9. Verfahren nach Anspruch 7 oder 8, worin die Ammonium-verbindung Ammoniumcarbonat ist.

10. Verfahren zur Herstellung einer Verbindung der Formel I gemäß der Definition in Anspruch 1, wobei ein Aldehyd der Formel II

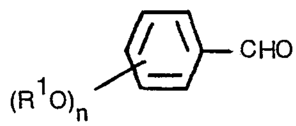

(II)

worin $R^1$ und n der in Anspruch 1 angegebenen Bedeutung entsprechen, mit einer Verbindung der Formel $R^2$—CH(SH)—$CONH_2$, worin $R^2$ der in Anspruch 1 angegebenen Definition entspricht, in einem inerten Lösungsmittel umgesetzt wird.

11. Verfahren nach Anspruch 10, worin die Reaktion bei einer temperatur von 50 bis 150°C durchgeführt wird.

12. Pharmazeutische Zusammensetzung, die eine Verbindung der Formel I nach einem der Ansprüche 1 bis 6 enthält oder die eine Verbindung enthält, welche nach einem Verfahren gemäß einem der Ansprüche 7 bis 11 hergestellt wurde, als aktiven Bestandteil zusammen mit einem pharmazeutisch unbedenklichen Träger.

13. Pharmazeutische Zusammensetzung zur Behandlung von Magengeschwüren, welche in Einheitsdosierungsform eine therapeutisch wirksame Menge einer Verbindung nach formel I gemäß der Definition in Anspruch 1 und einen pharmazeutisch unbedenklichen Träger enthält.

**Revendications**

1. Un composé consistant en (phényle substitué)-2-alkylthiazolidine-5-one-4 présentant la formule (I):

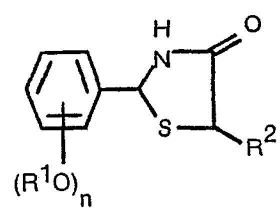

(I)

dans laquelle:

$R^1$ et $R^2$ indépendamment l'un de l'autre, représentent un radical alkyle comprenant 1 à 3 atomes de carbone; et n est un nombre entier de 1 à 3.

2. Un composé selon la revendication 1, présentant la formule:

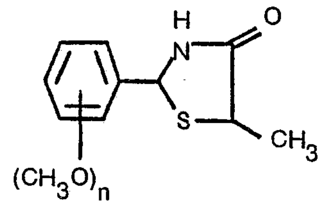

dans laquelle:

n est un nombre entier de 1 à 3.

3. Un composé selon la revendication 1, consistant en (diméthoxy-3,4-phényl)-2-méthylthiazolidine-5-one-4.

4. Un composé selon la revendication 1, consistant en (triméthoxy-3,4,5-phényl)-2-méthylthiazolidine-5-one-4.

5. Un composé selon la revendication 1, consistant en (méthoxy-2-phényl)-2-méthylthiazolidine-5-one-4.

6. Un composé selon la revendication 1, consistant en (méthoxy-4-phényl)-2-méthylthiazolidine-5-one-4.

7. Un procédé de production d'un composé de formule (I) tel que défini dans la revendication 1, ce procédé consistant à faire réagir un aldéhyde représenté par la formule (II):

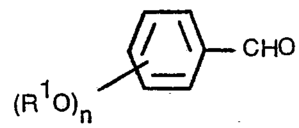
(II)

dans laquelle:

$R^1$ et n sont tels que définis dans la revendication 1, avec un composé de formule:

$$R^2—CH(SH)—COR^3$$

dans laquelle:

$R^2$ est tel que défini dans la revendication 1; et $R^2$ représente un groupe hydroxyle ou un radical alkoxy comprenant 1 ou 2 atomes de carbone, et un composé à base d'ammonium dans un solvant inerte.

8. Un procédé selon la revendication 7, dans lequel la réaction est mise en oeuvre à une température comprise entre 50 et 150°C.

9. Un procédé selon la revendication 7 ou 8, dans lequel le composé à base d'ammonium et le carbonate d'ammonium.

10. Un procédé de préparation d'un composé de formule (I) tel que défini dans la revendication 1, ce procédé comprenant la réaction d'un aldéhyde représenté par la formule (II):

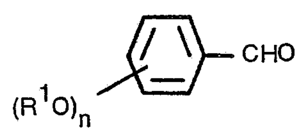
(II)

dans laquelle:

$R^1$ et n sont tels que définis dans la revendication 1, avec un composé représenté par la formule:

$$R^2—CH(SH)—CONH_2$$

dans laquelle:

$R^2$ est tel que défini dans la revendication 1, dans un solvant inerte.

11. Un procédé selon la revendication 10, dans lequel la réaction est mise en oeuvre à une température comprise entre 50 et 150°C.

12. Une composition pharmaceutique comprenant un composé de formule (I) ainsi que revendiqué dans l'une quelconque des revendications 1 à 6 ou ayant été produit par un procédé ainsi que revendiqué dans l'une quelconque des revendications 7 à 11, constituant l'ingrédient actif, associé à un support acceptable du point de vue pharmaceutique.

13. Une composition pharmaceutique convenant au traitement d'ulcère peptique, ladite composition comprenant une forme de dose unitaire consistant en une quantité efficace du point de vue thérapeutique d'un composé de formule (I) tel que défini dans la revendication 1, et un support acceptable du point de vue pharmaceutique.